# EUROPÄISCHE PATENTSCHRIFT

(11) **EP 0 882 436 B1**
(45) Veröffentlichungstag und Bekanntmachung des Hinweises auf die Patenterteilung: **21.08.2002**
(21) Anmeldenummer: 97810352.1
(22) Anmeldetag: 05.06.1997
(51) Int. Cl.: A61F 2/46

(54) **Transport- und Verarbeitungsvorrichtung für Zweikomponentenmaterial**
Transport and process device for two-component material
Dispositif de transport et de traitement pour un matériau à deux composants

(43) Veröffentlichungstag der Anmeldung: 09.12.1998
(73) Patentinhaber: Sulzer Orthopädie AG, 6340 Baar (CH)
(72) Erfinder: Heller, Mathias, 8404 Winterthur (CH); Spaltenstein, Anton, 8302 Kloten (CH); Dubach, Werner Fritz, 8124 Maur (CH)
(74) Vertreter: Manitz, Finsterwald & Partner Gbr

(56) Entgegenhaltungen:
- EP-A- 0 397 589
- WO-A-87/05492

## Beschreibung

Die Erfindung handelt von einer Transport- und Verarbeitungsvorrichtung für Zweikomponentenmaterial, insbesondere für Knochenzement, mit einer Flüssigkomponente und mit einer Pulverkomponente die durch eine Membran getrennt jeweils einen Transportraum in einem verschliessbaren Behälter einnehmen, welcher auf der einen Seite einen Ausstosskolben und auf der gegenüberliegenden Seite eine Oeffnung für das Ausstossen von gemischtem Zweikomponentenmaterial aufweist, und welcher einen perforierten Mischkolben einschliesst.

Eine Vorrichtung mit einer Flüssigkomponente und mit einer Pulverkomponente die durch eine Membran getrennt sind, ist aus der EP-A-397 589 bekannt.

Eine solche Vorrichtung ist in der EP-A-0 692 229 gezeigt. Zwischen einem Austragskolben und einem Mischkolben ist ein Trennkolben angeordnet, der den pulverförmigen Bereich vom Bereich der Flüssigkomponente trennt. Der Mischzylinder setzt sich aus zwei getrennten Transportzylindern zusammen, die über eine Schnellkupplung zusammensetzbar sind. Dies hat den Nachteil, dass Mischkolben, Trennkolben und Ausstosskolben während ihrer Betätigung über eine Trennstelle zwischen den Transportzylindern laufen müssen, welche eine Unstetigkeit darstellt.

Aufgabe der vorliegenden Erfindung ist es, eine sichere und funktionstüchtige Transport- und Verarbeitungsvorrichtung zu schaffen.

Diese Aufgabe wird mit den Kennzeichen vom unabhängigen Anspruch 1 dadurch gelöst, dass der Transportraum für die Flüssigkomponente ringförmig mit einem mittleren Durchbruch und mit einer Membran ausgeführt ist, dass gemischtes Zweikomponentenmaterial durch den mittleren Durchbruch und die Oeffnung ausstossbar ist und dass durch eine Relativbewegung zwischen dem Transportraum der Flüssigkomponente und eines im Behälter eingeschlossenen festen Körpers, die Membran zerstörbar ist, um ein Fliessen der Flüssigkomponente in den Transportraum der Pulverkomponente zu bewirken.

Ein Vorteil dieser Vorrichtung ist, dass sie ohne gummiähnliche Weichdichtungen in Form von O-Ringen oder Lippen ausführbar ist. Dies gestattet es, die Vorrichtung bis auf eine Membran aus Kunststoffspritzteilen herzustellen. Im weiteren müssen keine kollabierten Transportbehälter beim Ausstossen der Mischung durch den Ausstosskolben mitbewegt werden.

Ein weiterer Vorteil dieser Vorrichtung ist, dass vormontierte Kunststoffspritzteile an ein Abfüllwerk lieferbar sind und dass mit dem Abfüllen eine endgültige Montage, ein Einschweissen und wo notwendig ein Sterilisieren möglich ist. Unabhängig von Transport und Lagerung gelangen unter kontrollierten Bedingungen abgefüllte Mengen mit der betriebsbereiten Vorrichtung an den unmittelbaren Arbeitsort. Zum Mischen und Ausstossen der beiden Komponenten kann die Vorrichtung äusserlich betätigt werden. Der Innenraum ist bis zum Anstossen verschliessbar.

Vorteilhafte Weiterbildungen der Erfindung sind in den abhängigen Ansprüchen 2 bis 19 aufgeführt.

Durch ein Ausbilden der Membran als ringförmige Folie, kann der Transportraum der Flüssigkomponente in einem einfachen Spritzwerkzeug hergestellt werden und ist eine Angriffskraft zum Zerstören der Membran überall auf dem Querschnitt des Transportraumes wirksam. Eine von aussen verschliessbare Abfüllöffnung am Transportraum der Flüssigkomponente gestattet es, Flüssigkomponente und Pulverkomponente in der gleichen vertikalen Stellung des Behälters abzufüllen. Durch eine axiale Fixiermöglichkeit für den Mischkolben während Transport und Lagerung kann beim Mischen die Bewegung des Mischkolbens zum Zerstören der Membran genutzt werden. Ein Verdrängerkörper, der in den Transportraum der Flüssigkomponente eindringt, hat den Vorteil, dass die Flüssigkomponente zwangsläufig und unabhängig von der Schwerkraft ausgestossen wird. Der Verdrängerkörper kann mit einer Schneide versehen werden und so die Membran mit dem Beginn der Relativbewegung zwischen Verdrängerkörper und Transportraum der Flüssigkeit zerstören. Wenn sich Verdrängerkörper und Transportraum am Ende der Ausstossbewegung auch noch gegenseitig blockieren, wird dieser Vorgang irreversibel und es besteht keine Gefahr, dass unterschiedlich gemischte Produktteile in den Transportraum der Flüssigkomponente zurückgestossen werden. Mit einer hohlen Kolbenstange für den Mischkolben, die einen bis zum Kolben reichenden Verschluss aufweist, kann die Kolbenstange als Austrittsrohr genutzt werden, ohne dass Toträume mit ungleicher Mischung entstehen.

Eine Ausführung des Transportraumes der Flüssigkomponente als Ringraum gestattet es auch, den Transportraum in einen Behälterdeckel zu integrieren, der gegenüber dem Behälter axial beweglich ist, um die Membran an einer ortsfesten Schneide im Behälter zu zerstören und um die Flüssigkomponente unter Schwerkraft in den darunterliegenden Transportraum der Pulverkomponente fliessen zu lassen. Von Vorteil ist dabei ein Ableitring, über den die Flüssigkomponente abfliesst und ein Schleppring, der sich beim ersten axialen Absenken des Behälterdeckels mit diesem verklinkt und beim Anheben des Behälterdeckels einen Abflussquerschnitt freigibt. Bei einem erneuten Absenken des Behälterdeckels wird dieser Abflussquerschnitt wieder verschlossen, um während dem anschliessenden Mischen und Ausstossen der beiden Komponenten zu verhindern, dass Knochenzement in den Transportraum der Flüssigkomponente gelangt. Eine von aussen steuerbare Axialbewegung kann zum Beispiel durch einen Behälterdeckel mit einer zum Behälter schraubenförmig geführten Drehbewegung erzeugt werden.

Im folgenden wird die Erfindung anhand von Ausführungsbeispielen beschrieben. Es zeigen:
- Fig. 1: Schematisch einen Längsschnitt durch eine abgefüllte Transport- und Verarbeitungsvorrichtung, bei der der Transportraum für die Flüssigkomponente in den Behälterdeckel integriert ist, und die einen Verdrängerkörper besitzt;
- Fig. 2: schematisch im Längsschnitt von Fig. 1 nach dem Mischen den Transportraum der Flüssigkomponente mit einem eingefahrenen und blockierten Verdrängerkörper;
- Fig. 3: schematisch im Längsschnitt eine abgefüllte Transport- und Verarbeitungsvorrichtung, bei der ein Verdrängerkörper in den Behälterdeckel integriert ist, während der Transportraum der Flüssigkomponente als separates Gefäss in einer Transportstellung im Behälter fixiert ist;
- Fig. 4: schematisch im Längsschnitt von Fig. 3 nach dem Mischen den eingefahrenen und blockierten Transportraum der Flüssigkomponente;
- Fig. 5: schematisch im Längsschnitt eine Anordnung analog zu Fig. 1, bei der ein Mischkolben durch den Ausstosskolben hindurch mit einer Kolbenstange betätigbar ist;
- Fig. 6: schematisch im Längsschnitt die Anordnung von Fig. 5 nach dem Mischen;
- Fig. 7a, b, c: schematisch im Längsschnitt das Einfüllen von Flüssigkomponente und Pulverkomponente in einer Anordnung nach Fig. 5;
- Fig. 7d: schematisch im Längsschnitt eine abgefüllte und gebrauchsfertige Vorrichtung nach Fig. 5;
- Fig. 7e: schematisch im Längsschnitt zu Fig. 5 das Zusammenführen von Flüssig- und Pulverkomponente;
- Fig. 7f: schematisch im Längsschnitt zu Fig. 5 das Mischen der beiden Komponenten;
- Fig. 7g: schematisch im Längsschnitt zu Fig. 5 ein Ablösen der Kolbenstange vom Mischkolben;
- Fig. 7h: schematisch im Längsschnitt zu Fig. 5 ein Absaugen von Dämpfen aus dem Behälter;
- Fig. 7i: schematisch im Längsschnitt zu Fig. 5 die ausstossfertige Mischung im Behälter;
- Fig. 8: schematisch im Längsschnitt eine Transport- und Mischvorrichtung mit axial beweglichem Deckel, welcher die Flüssigkomponente enthält, in Transportstellung;
- Fig. 9: schematisch im Längsschnitt die Vorrichtung von Fig. 8 nach dem Mischen der Komponenten, wobei die Kolbenstange vom Mischkolben bereits entfernt ist und stattdessen ein Ausstossrohr aufgesetzt ist;
- Fig. 10a, b, c: schematisch im Längsschnitt das Abfüllen der Komponenten in einer Vorrichtung nach Fig. 8;
- Fig. 10d: schematisch im Längsschnitt eine gebrauchsfertige Vorrichtung nach Fig. 8; und
- Fig. 10e, f, g, h, i, j: schematisch im Längsschnitt von Fig. 8 das Zusammenführen und Mischen der Komponenten, das Absaugen von Lösungsmitteldämpfen und eine ausstossbereite Vorrichtung mit aufgesetztem Ausstossrohr.

Die Figuren zeigen Transport- und Verarbeitungsvorrichtungen für Zweikomponentenmaterial, insbesondere für Knochenzement, mit einer Flüssigkomponente und mit einer Pulverkomponente, die durch eine Membran getrennt jeweils einen Transportraum in einem verschliessbaren Behälter einnehmen, welcher auf der einen Seite einen Ausstosskolben und auf der gegenüberliegenden Seite eine Oeffnung für das Ausstossen von gemischtem Zweikomponentenmaterial aufweist. Durch ringförmige Gestaltung des Transportraumes der Flüssigkomponente mit einem mittleren Durchbruch kann gemischtes Zweikomponentenmaterial durch den Durchbruch ausgestossen werden. Durch eine Relativbewegung zwischen dem Transportraum der Flüssigkomponente und eines im Behälter eingeschlossenen festen Körpers wird die Membran zerstört, um ein Fliessen der Flüssigkomponente in den Transportraum der Pulverkomponente zu bewirken und das Mischen mit einem Mischkolben zu ermöglichen.

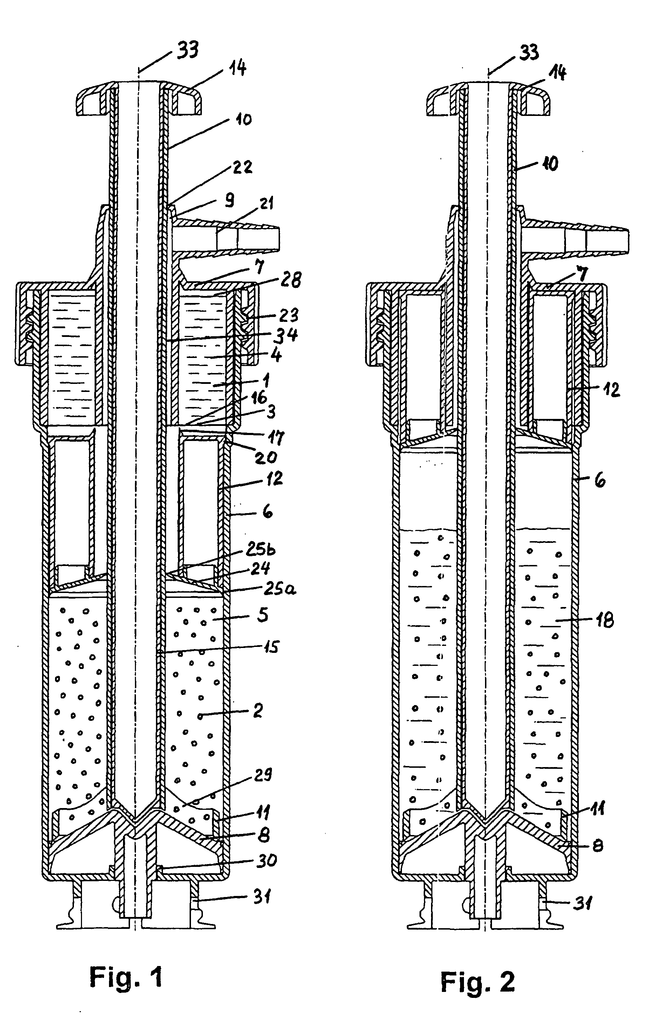

Im Beispiel von Fig. 1 und 2 besteht der Transportbehälter aus einem eigentlichen Behälter 6 und aus einem Behälterdeckel 7 der als Transportraum 4 für die Flüssigkomponente 1 ausgeführt ist, welcher Ringform hat und welcher mit einer Membran 3 in Form einer Kreisringfolie 16 verschlossen ist. Das Abfüllen der Flüssigkomponente 1 und Versiegeln des Transportraums 4 mit der Folie 16 geschieht in einer Voroperation. Zum weiteren Konfektionieren der Einrichtung wird:
- Der Ausstosskolben 8 im zylindrischen Behälter 6 eingepresst und mit einer Schnappverbindung 30 fixiert;
- der Mischkolben 11 mit Kolbenstange 10 auf dem Ausstosskolben 8 abgestellt;
- die Pulverkomponente 2 eingefüllt;
- der Verdrängerkörper 12 in den Behälter 6 eingesetzt und verklinkt, wobei der Verdrängerkörper 12 eine Abdeckung 24 mit zwei Führungslippen 25a, b besitzt, die die Pulverkomponente 2 zurückhalten;
- der Deckel 7 mit der Flüssigkomponente 1 im Transportraum 4 über die hohle Kolbenstange 10 geführt und mit einer Verschraubung 23 am Behälter 6 befestigt;
- ein Verschluss 14 in Form einer Ausbuchtung in die Kolbenstange eingeführt und der Behälter 6 verschlossen, wobei einzig eine Absaugöffnung 21 noch nicht verschlossen ist.

In diesem Zustand wäre die Vorrichtung gebrauchsfähig zum Mischen der beiden Komponenten. Bei Knochenzement wird die Einrichtung für Transport und Lagerung in eine Folie eingeschweisst und mit γ-Strahlen oder Gas, z.B. Ethylenoxid oder Formaldehyd sterilisiert. Die Teile der Vorrichtung sind als Spritzgussteile aus Kunststoff ausgeführt und transparent, um den Einfüllvorgang und später das Mischen überwachen zu können. Das Sterilisieren geschieht entweder vor dem Abfüllen mit γ-Strahlung, oder nachher mit Gas, damit die Flüssigkeit keinen Schaden durch γ-Strahlen nimmt.

Bei Knochenzement wird die Vorrichtung steril in Operationsraum gebracht. Zum Mischen wird:
- Der Mischkolben 11 mit der Kolbenstange 10 aus einer Transportsicherung 22 heraus und durch die Pulverkomponente 2 hindurchgezogen, wobei das fliessfähige Pulver durch Schlitze 29 an Mischkolben 11 vorbeifliesst;
- der Verdrängerkörper 12 mit seiner Schneide 17 durch den Mischkolben 11 aus seiner Schnappverbindung 20 herausgerissen und in den Transportraum 4 der Flüssigkomponente gepresst, wobei die Schneide 17 die Folie 16 aufreisst und der Verdrängerkörper 12 die Flüssigkomponente 1 vollständig in den Transportraum 5 der Pulverkomponente verdrängt;
- der Verdrängerkörper 12 in seiner Endstellung (Fig. 2) im Transportraum 5 der Flüssigkomponente 1 blockiert, sodass nur noch Komponenten 1, 2 und Mischkolben 11 gegeneinander beweglich sind;
- der Mischkolben unter Drehung eine vorgesehene Anzahl Hübe, beispielsweise 30 Hübe, auf und ab gestossen, um ein Durchmischen der Komponenten 1, 2 zu erreichen;
- an der Absaugöffnung befristet ein Vakuum angesetzt, um Dämpfe und aufsteigende Luftblasen abzusaugen; und
- der Verschluss 14 abgenommen, der Mischkolben 11 in einer oberen Position gehalten und ein Vorschubapparat mit Schubstange an einem Bajonettverschluss 31 aufgesetzt, um die fertige Mischung 18 mit dem Ausstosskolben 8 durch die hohle Kolbenstange 10 auszustossen.

Verschlussstopfen 14 und sein Handgriff lassen sich von der Kolbenstange 10 abschrauben. Dadurch, dass die meisten Teile zur Hauptachse 33 weitgehend rotationssymmetrisch ausgeführt sind, ergeben sich günstige Spritzformen.

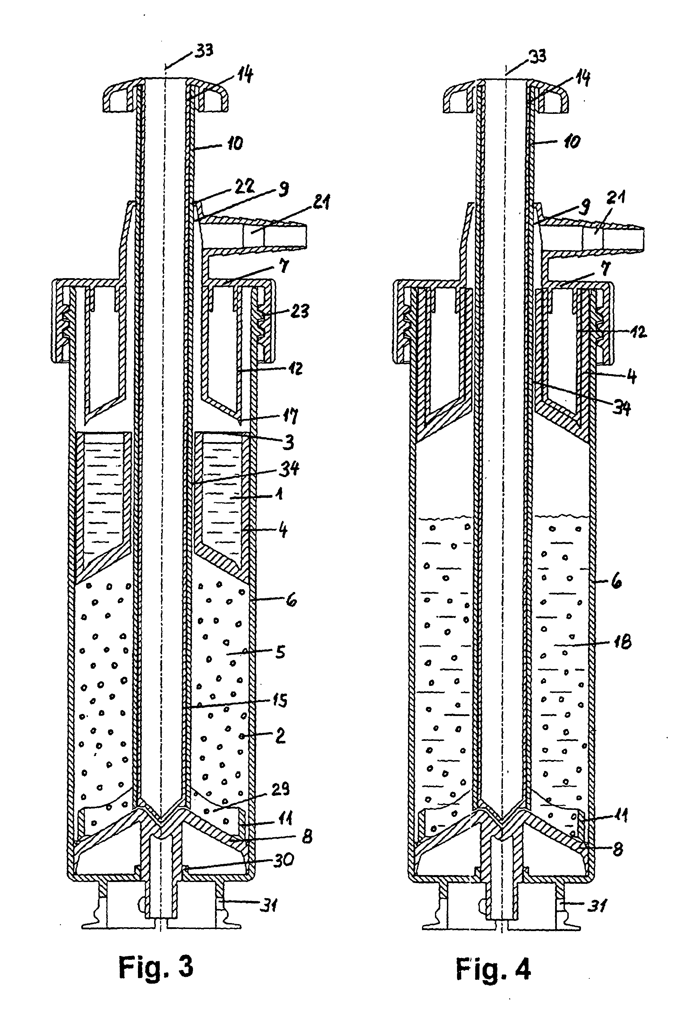

Im Beispiel von Fig. 3, 4 sind gegenüber dem vorhergehenden Beispiel lediglich die Anordnung von Verdrängerkörper 12 und des Transportraums 4 der Flüssigkomponente 1 vertauscht. Behälter 6 und Ausstosskolben 8 werden ebenfalls vormontiert. Der Transportraum 4 für die Flüssigkomponente 1 ist ein separater Behälter, der mit der Flüssigkomponente 1 gefüllt und mit einer Folie 3 geschlossen und verschweisst wird. Zum Befüllen der Vorrichtung werden Mischkolben 11 und Kolbenstange 10 im Behälter 6 eingesetzt. Anschliessend wird die Pulverkomponente 2 eingefüllt und der ringförmige Transportraum 4 der Flüssigkomponente über die Kolbenstange 10 geschoben und im Behälter 6 verankert. Der Deckel 7 mit dem integrierten Verdrängerkörper 12 wird aufgesetzt und verschliesst den Behälter 6 mit der Verschraubung 23. Als letztes wird der Verschluss 14, der einen Handgriff und eine Ausstülpung bis zum Mischkolben 11 aufweist, mit der rohrförmigen Kolbenstange 10 über ein Gewinde beim Handgriff verbunden. Die Ausstülpung 15 verhindert, dass beim nachfolgenden Mischen, Toträume mit ungleicher Verteilung der Komponenten 1, 2 entstehen.

Zum eigentlichen Mischen Fig. 4 durchquert: der Mischkolben 11 die Pulverkomponente 2 und stösst den Transportraum 4 der Flüssigkomponente in die Schneide 17 zum Zerstören der Membran 3 und weiter über den Verdrängerkörper 12, um die Flüssigkomponente 1 auszustossen. In ihrer Endstellung blockieren sich Verdrängerkörper 12 und der Transportraum 4. Nach dem Mischen mit dem Mischkolben 11 und dem eventuellen Absaugen von Dämpfen über eine Absaugöffnung 21 wird der Mischkolben 11 in einer oberen Stellung über die Kolbenstange 10 fixiert und der Verschluss 14 mit Handgriff und Ausstülpung 15 wird abgenommen. Nach dem Ansetzen eines Vorschubapparates (hier nicht gezeigt) am Bajonettverschluss 31 kann der gemischte Zement 18 durch die konzentrisch zueinanderliegenden Oeffnungen 9, 34, 10 ausgestossen werden.

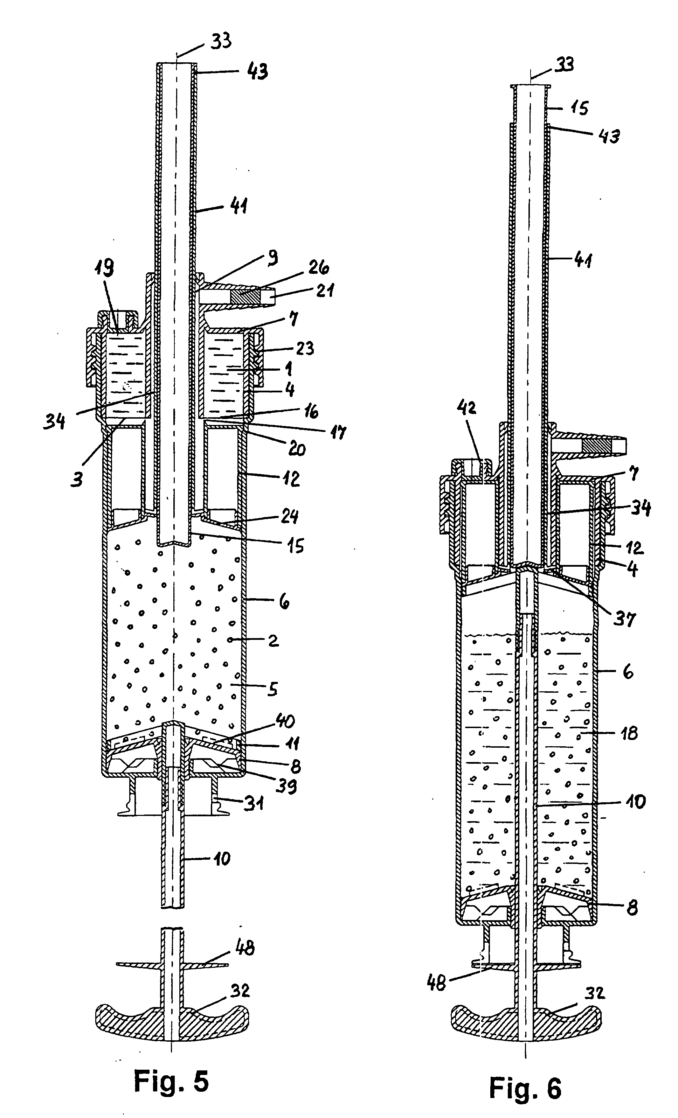

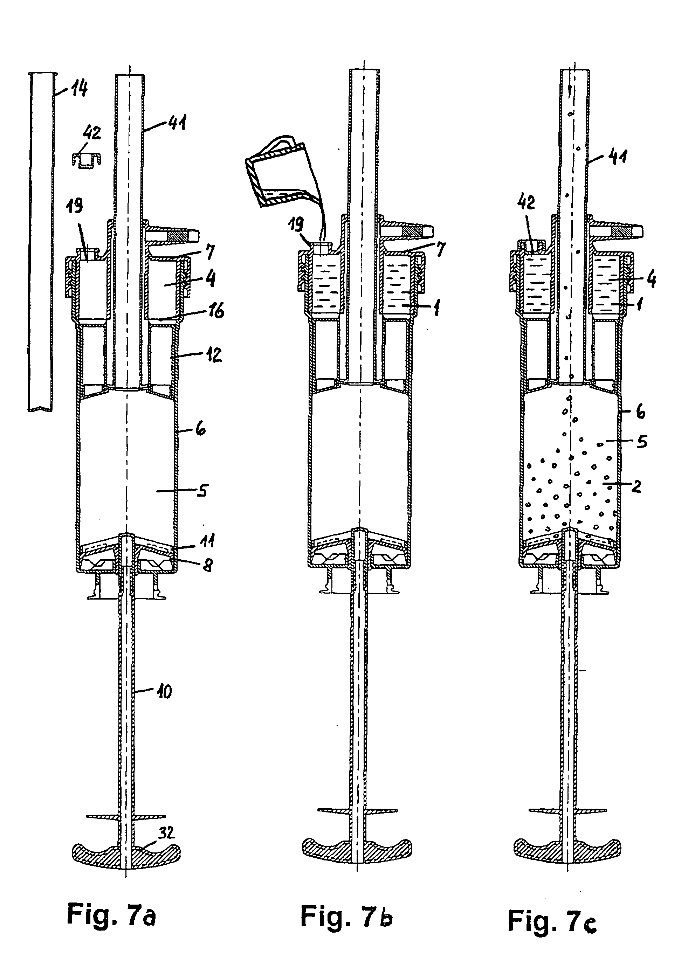

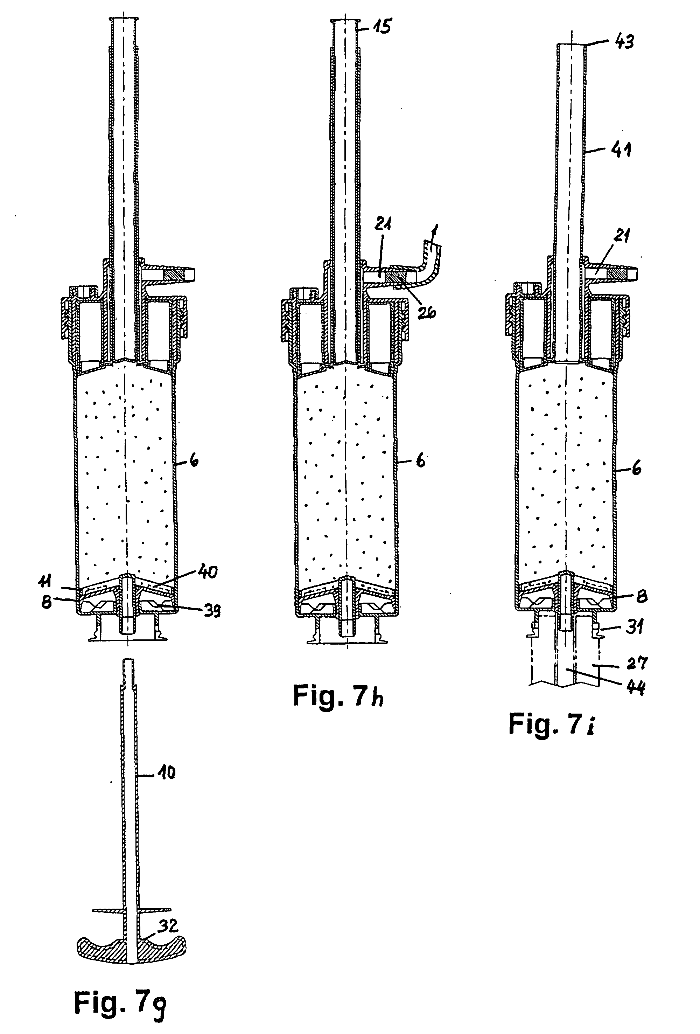

Eine weitere Konstruktion ist in den Fig. 5, 6 und 7a bis 7i gezeigt. Die Vorrichtung besteht (Fig. 5, 6) aus einer zu einer Längsachse 33 konzentrischen Anordnung mit einem Behälter 6, der auf seiner Unterseite eine Oeffnung für eine Nabe vom Ausstosskolben 8 aufweist und über einen Bajonettverschluss 31 mit einem Vorschubapparat 27 (Fig. 7i) verbindbar ist. Auf seiner Oberseite ist der Behälter 6 über eine Verschraubung 23 mit einem Deckel 7 verschlossen, welcher eine Oeffnung 9 besitzt, von der eine Absaugöffnung 21 mit Filter 26 abzweigt. Der Deckel 7 ist gleichzeitig als Transportraum 4 für die Flüssigkomponente 1 ausgeführt. An seiner Unterseite ist er mit einer Membran 3 in Form einer ringförmigen Folie 16 verschlossen. An seiner Oberseite ist eine nicht zentrische Einfüllöffnung 19 angebracht, die mit einem Stopfen 42 verschliessbar ist. Ein Verdrängerkörper 12 ist in einer Transportstellung mit dem Behälter 6 über eine Schnappverbindung 20 verbunden und besitzt ein Ausflussrohr 41, welches durch die Oeffnung 9 im Deckel 7 herausgeführt ist. Das Ausflussrohr 41 ist mit einer Ausstülpung 15 verschliessbar. Am Uebergang zwischen Ausflussrohr und eigentlichem Verdrängerkörper 12 sind Ausflussöffnungen 37 angebracht, die freigegeben werden, wenn ein Mischkolben 11 die oberste Stellung gegenüber dem Verdrängerkörper 12 erreicht und die Ausstülpung 15 zurückstösst. Konstruktiv ist das Ausflussrohr 41 ein Teil von einer Abdeckung 24, die den hohlen Verdrängerkörper 12 verschliesst. Der Mischkolben 11 ist in seiner unteren Stellung mit dem Ausstosskolben 8 über eine Verklinkung 40 verbindbar, wobei der Ausstosskolben 8 in seiner unteren Stellung über eine Drehsicherung 39 zum Behälter 6 Drehmomente aufnehmen kann. Der Ausstosskolben 8 besitzt an seiner Nabe eine zentrale Bohrung durch die ein Stück der Kolbenstange 10 für den Mischkolben 11 in Form einer Hülse mit Gewinde herausgeführt ist. Die eigentliche Kolbenstange 10 besitzt einen Handgriff 32 und einen Anschlag 48. Sie ist über ein Gewinde mit dem Mischkolben 11 verschraubbar.

Fig. 7a zeigt die Vorrichtung im vormontierten Zustand. Der Ausstosskolben 8 ist eingesetzt. Der Mischkolben 11 ist in der untersten Stellung mit dem Ausstosskolben 8 verklinkt und seine Kolbenstange 10 ist eingesetzt. Der Verdrängerkörper 12 mit Ausflussrohr 41 ist mit dem Behälter 6 in Transportstellung verbunden. Der Deckel 7 ist mit dem Behälter 6 verschraubt.

In Fig. 7b wird durch eine Abfüllöffnung 19 im Deckel 7 die Flüssigkomponente 1 eingefüllt und die Abfüllöffnung mit einem Stopfen 42 verschlossen. Für grössere Serien erfolgt das Abfüllen an einer Abfüllstation mit Tauchsonde und vorportionierten Flüssigkeitsmengen.

In Fig. 7c wird die Pulverkomponente 2 in ihren Transportraum 5 im Behälter 6 durch das Ausflussrohr 41 eingefüllt. Um die Ausflussöffnungen 3 am Verdrängerkörper 12 nicht zuzusetzen und um Staubentwicklungen zu vermeiden, ist eine Tauchsonde, die zum Abfüllen in den Transportraum 5 ragt, vorteilhaft.

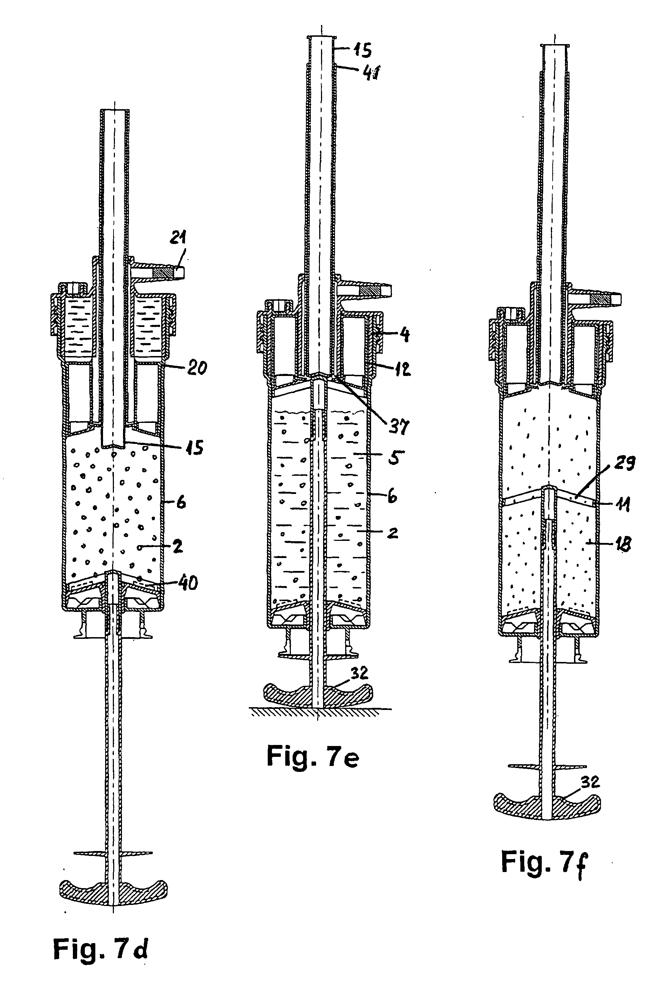

In Fig. 7d ist der gefüllte Behälter 6 mit der Ausstülpung 15 verschlossen und kann zum Beispiel für Knochenzement in dieser Anordnung in einer Folie eingeschweisst und sterilisiert werden. Die Vorrichtung ist in dieser Anordnung transportierbar und lagerbar und jederzeit für ein Mischen und Ausstossen vom Zweikomponentenmaterial 1, 2 betriebsbereit.

Zum Mischen wird die Vorrichtung mit dem abwärtsgerichteten Handgriff 32 auf eine Gegenfläche gepresst, um die Verklinkung 40 zwischen Mischkolben und Ausstosskolben zu lösen. Der Mischkolben durchquert die Pulverkomponente 2 und trifft auf der Ausstülpung 15 auf, die soweit gegen aussen herausgestossen wird (Fig. 7e), dass die Ausflussöffnungen 37 für die Flüssigkomponente 1 freigelegt werden. Gleichzeitig wird der Verdrängerkörper 12 aus seiner Schnappverbindung 20 gerissen und zerstört mit seiner Schneide die Folie des Transportraums 4 der Flüssigkomponente, um die Flüssigkomponente 1 vollständig zu verdrängen. Diese fliesst durch die Ausflussöffnungen 37 in den Transportraum 5 der Pulverkomponente. Verdrängerkörper 12 und Ausflussrohr 41 blockieren sich in einer obersten Stellung und das eigentliche Durchmischen (Fig. 7f) kann stattfinden. Dazu wird der Mischkolben 11 unter Drehung über eine vorgegebene Anzahl Hübe auf und ab bewegt und beide Komponenten werden unter gegenseitiger Reibung durch Schlitze 29 am Mischkolben 11 gepresst.

In Fig. 7g wurde der Mischkolben 11 in der untersten Stellung am Ausstosskolben verklinkt 40, um die Kolbenstange 10 mit dem Handgriff 32 vom Mischkolben 11 abzuschrauben. Das dazu notwendige Gegenmoment wird vom Behälter 6 über die Drehsicherung 39 auf den Ausstosskolben 8 und von diesem über die Verklinkung 40 auf den Mischkolben übertragen.

In Fig. 7h ist an der Absaugöffnung 21 ein Schlauch zum Absaugen von Lösungsmitteldämpfen angesetzt, wobei ein Filter 26 ein Vordringen von festen oder zähflüssigen Partikeln verhindert.

In Fig. 7i wurde ein nicht weiter gezeigter Vorschubapparat 27 am Bajonettverschluss 31 angesetzt. Dieser wirkt mit einer Schubstange 44 auf den Ausstosskolben 8. Sobald die Ausstülpung 15 vollständig entfernt ist, kann der noch flüssige Zement über eine Mündung 43 vom Ausflussrohr 41 ausgepresst werden.

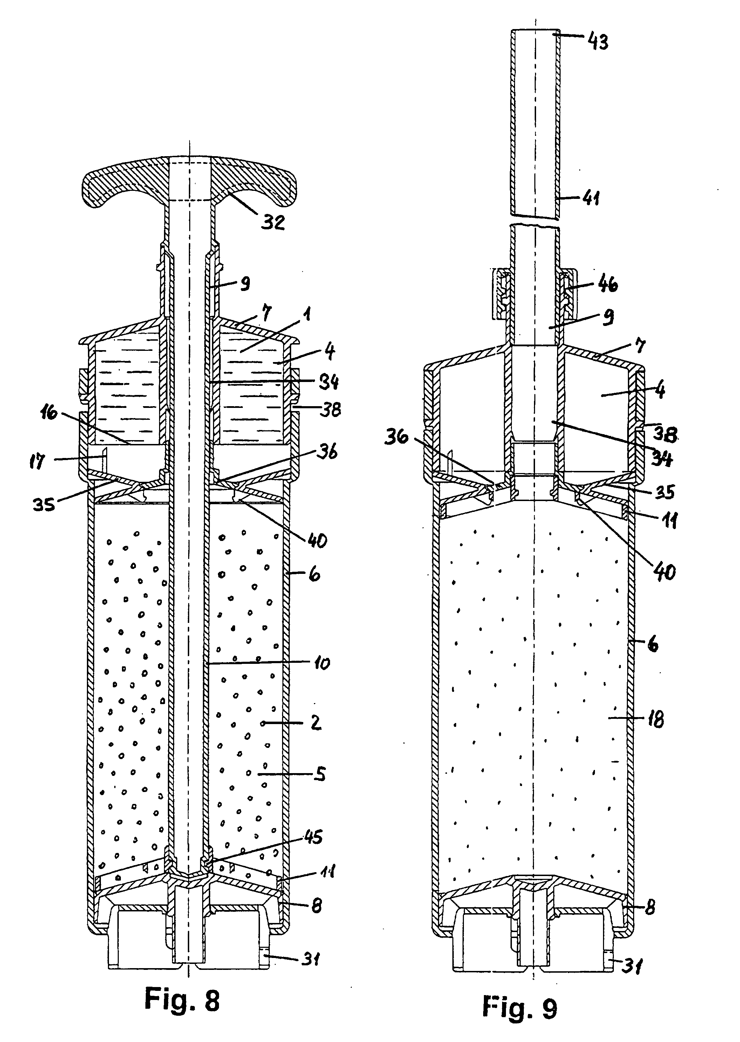

Ein weiteres Beispiel ist in den Fig. 8, 9 und 10a bis j gezeigt. In Fig. 8 ist eine betriebsbereite Vorrichtung dargestellt. Der Behälter 6 ist auf der Unterseite durch den Ausstosskolben 8 verschlossen, auf welchem der Mischkolben 11 aufliegt. Der Mischkolben 11 wird über eine Kolbenstange 10 betätigt, die eine lösbare Verbindung 45 zum Mischkolben 11 aufweist und die nach oben aus dem Behälterdeckel 7 herausgeführt ist und mit einem Handgriff 32 abschliesst. Der Transportraum 5 für die Pulverkomponente 2 wird nach oben durch einen eingepressten Ableitring 35 und durch einen Schleppring 36 abgeschlossen. Am ortsfesten Ableitring 35 sind einige, zum Beispiel vier, Schneiden 17 angebracht und eine Verklinkung 40 für den Mischkolben 11. Der Schleppring 36 kann nur unter Ueberwindung einer Klemmkraft in axialer Richtung bewegt werden. Er blockiert sich mit seiner obersten Stellung im Deckel 7 und ist dann nur noch mit dem Deckel bewegbar. Der Deckel 7 besitzt einen mittleren Durchbruch 34, durch den die Kolbenstange 10 einsteckbar und ausziehbar ist. Nach dem Mischen und dem Ausziehen der Kolbenstange kann der Zement durch diesen mittleren Durchbruch 34 und die daran anschliessende Oeffnung 9 ausgestossen werden. Der Deckel 7 ist als ringförmiger Transportraum 4 für die Flüssigkomponente 1 ausgeführt und auf seiner Unterseite mit einer Folie 16 verschlossen. Der Deckel ist in radialer Richtung in Aussparungen in Form einer Wendel 38 gelagert und kann um etwa 60° vorwärts und rückwärts gegenüber dem Behälter 6 gedreht werden, wobei er einen Hub von etwa 6 mm ausführt.

In Fig. 9 ist der Zement 18 bereits gemischt. Der Mischkolben 11 ist in seiner obersten Position über die Verklinkung 40 mit dem Ableitring 35 verbunden. Die Kolbenstange 10 ist durch Lösen der Verbindung 45 ausgezogen worden und stattdessen ist ein Ausflussrohr 41 mit Mündung 43 über eine Kupplung 46 am Deckel 7 angesetzt worden. Der Deckel 7 befindet sich in seiner unteren Position gegenüber dem Behälter 6 und sichert den Schleppring 36.

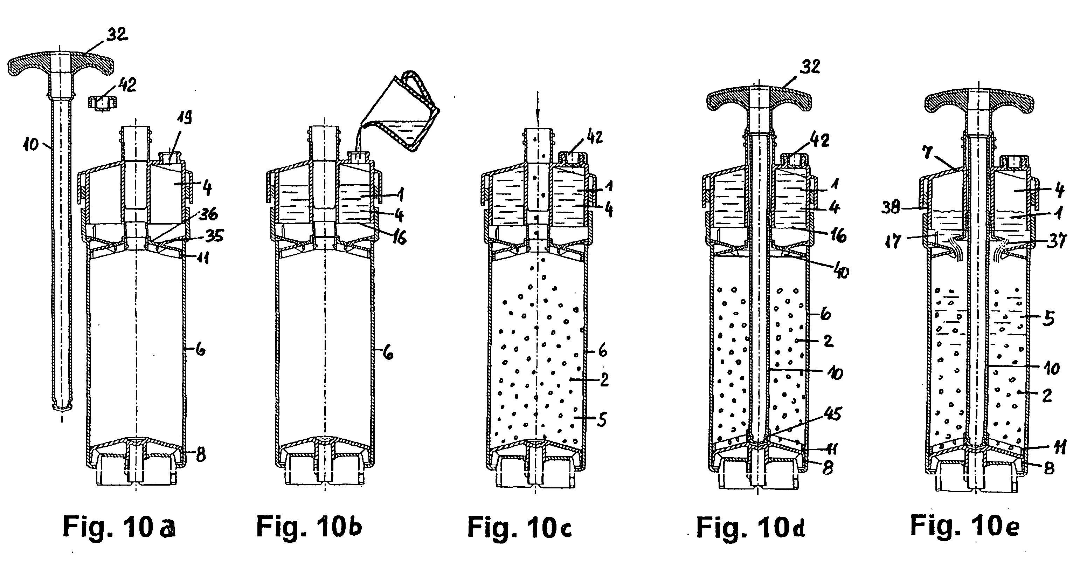

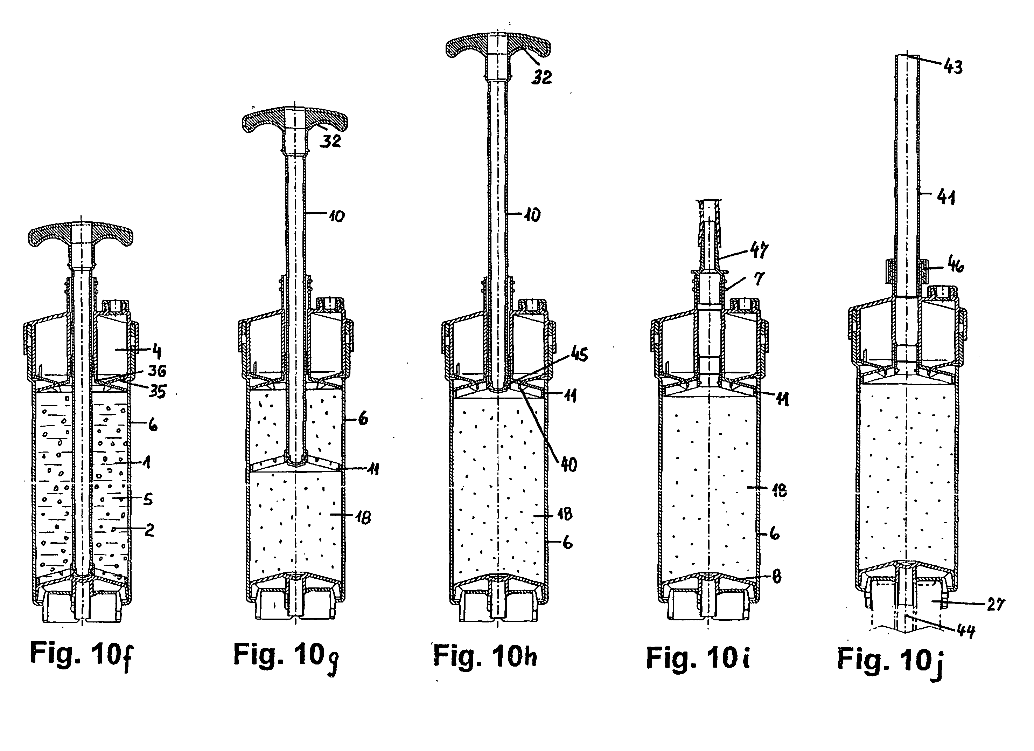

In Fig. 10a ist der Anlieferzustand der Vorrichtung vor dem Abfüllen gezeigt. Am Behälter 6 ist der Ausstosskolben 8 eingesetzt. Ebenso wurde der Ableitring 35 mit dem verklinkten Mischkolben 11 mit einer dosierten Kraft auf einer Presse eingepresst. Der Deckel 7 ist bereits mit einer Folie 16 versiegelt und mit dem eingesetzten Schleppring 36 in seiner oberen Stellung gegenüber dem Behälter 6 aufgesteckt worden und besitzt noch eine Oeffnung 19 zum Abfüllen. Verschlussstopfen 42 und Kolbenstange 10 mit Handgriff 32 werden lose mitgeliefert.

In Fig. 10b wird die Flüssigkomponente 1 in ihren Transportraum 4 abgefüllt. Eine Abfüllsonde ist auch hier von Vorteil. Allerdings darf sie die Verschlussfolie 16 nicht beschädigen.

In Fig. 10c ist der Transportraum 4 der Flüssigkomponente 1 bereits mit dem Stopfen 42 verschlossen und der Transportraum 5 der Pulverkomponente 2 wird aufgefüllt. Auch hier ist eine Tauchsonde zum Abfüllen von Vorteil.

In Fig. 10d wurde die Kolbenstange 10 in den abgefüllten Behälter 6 von oben eingesetzt und in ihrer tiefsten Position über einen Kragen mit dem Behälterdeckel 7 verbunden. Beim Abwärtsfahren wurde der Mischkolben aus seiner Verklinkung 40 gelöst und mitgenommen. Gleichzeitig wurde eine lösbare Verbindung 45 zur Kolbenstange 10 hergestellt. Das Gerät wäre jetzt für ein Mischen betriebsbereit und kann in dieser Form gelagert und transportiert werden.

In Fig. 10e wird die Flüssigkomponente 1 durch eine erste Vorwärtsdrehung und sofort anschliessende Rückwärtsdrehung des Deckels 7 freigesetzt. Beim Vorwärtsdrehen entlang der Wendel 38 taucht der Transportbehälter 4 mit der Folie 16 in die Schneiden 17 und wird aufgerissen. Gleichzeitig schiebt sich der Deckel 7 auf den Schleppring und hält diesen fest. Beim sofort anschliessenden Zurückdrehen hebt der Deckel 7 den Schleppring ab und gibt eine ringförmige Ausflussöffnung 37 für die Flüssigkomponente frei, welche nun unter Schwerkraft in den Transportraum 5 der Pulverkomponente 2 ausfliesst. Nach dem vollständigen Ausfliessen der Flüssigkomponente, was bei transparentem Behälterdeckel 7 kontrollierbar ist, erfolgt eine zweite Vorwärtsdrehung des Deckels 7, die den Schleppring 36 gegen den Ableitring 35 presst und so beide Komponenten 1, 2 im Transportraum 5 der Pulverkomponente 2 für das Mischen einschliesst (Fig. 10f).

In Fig. 10g findet das eigentliche Mischen über Handgriff 32, Kolbenstange 10 und den Mischkolben 11 statt. Nach Abschluss der vorgesehenen Hubbewegungen wird die Kolbenstange 10 ganz nach oben gezogen (Fig. 10h). Der Mischkolben 11 fährt wieder in die Verklinkung 40 am Ableitring 35 ein und die Kolbenstange 10 löst sich aus ihrer Verbindung 45 am Mischkolben 11. Fakultativ kann anschliessend ein Absaugrohr 47 am Deckel 7 aufgesetzt werden (Fig. 10i), um Lösungsmitteldämpfe und Luftblasen vom gemischten Zement 18 abzusaugen.

In Fig. 10j ist statt dem Absaugrohr 47 ein Ausflussrohr 41 mit einer Kupplung 46 befestigt. Auf der Unterseite ist ein Vorschubapparat 27 am Behälter befestigt worden, der mit einer Schubstange 44 den Zement über eine Mündung 43 am Ausflussrohr 41 ausstossen kann.

## Patentansprüche

1. Transport- und Verarbeitungsvorrichtung für Zweikomponentenmaterial, insbesondere für Knochenzement, mit einer Flüssigkomponente (1) und mit einer Pulverkomponente (2) die durch eine Membran (3) getrennt jeweils einen Transportraum (4, 5) in einem verschliessbaren Behälter (6, 7) einnehmen, welcher auf der einen Seite einen Ausstosskolben (8) und auf der gegenüberliegenden Seite eine Oeffnung (9) für das Ausstossen von gemischtem Zweikomponentenmaterial aufweist, und welcher einen perforierten Mischkolben (11) einschliesst, **dadurch gekennzeichnet, dass** der Transportraum (4) für die Flüssigkomponente (1) ringförmig mit einem mittleren Durchbruch (34) und mit einer Membran (3) ausgeführt ist, dass gemischtes Zweikomponentenmaterial durch den mittleren Durchbruch (34) und die Oeffnung (9) ausstossbar ist und dass durch eine Relativbewegung zwischen dem Transportraum (4) der Flüssigkomponente (1) und eines im Behälter (6) eingeschlossenen festen Körpers (12, 17), die Membran (3) zerstörbar ist, um ein Fliessen der Flüssigkomponente in den Transportraum (5) der Pulverkomponente (2) zu bewirken.

2. Vorrichtung nach Anspruch 1, **dadurch gekennzeichnet, dass** die Membran (3) durch eine ringförmige Folie (16) gebildet ist.

3. Vorrichtung nach einem der Ansprüche 1 oder 2, **dadurch gekennzeichnet, dass** der Transportraum (4) der Flüssigkomponente ausser der Membran (3, 16) eine weitere von aussen verschliessbare Abfüllöffnung (19) aufweist.

4. Vorrichtung nach einem der Ansprüche 1 bis 3, **dadurch gekennzeichnet, dass** der Mischkolben (11) in mindestens einer Stellung axial fixierbar ist.

5. Vorrichtung nach einem der Ansprüche 1 bis 4, **dadurch gekennzeichnet, dass** ein Verdrängerkörper (12) im Behälter (6, 7) eingeschlossen ist, der durch den Mischkolben (11) eine Relativbewegung gegenüber dem Transportraum (4) der Flüssigkomponente (1) erfährt, um die Membran (3, 16) zu zerstören und die Flüssigkomponente (1) aus ihrem Transportraum (4) zu verdrängen.

6. Vorrichtung nach Anspruch 5, **dadurch gekennzeichnet, dass** sich Verdrängerkörper (12) und Transportraum (4) der Flüssigkomponente (1) am Ende des Verdrängens der Flüssigkomponente (1) gegenseitig blockieren.

7. Vorrichtung nach einem der Ansprüche 1 bis 6, **dadurch gekennzeichnet, dass** der Mischkolben (11) eine Kolbenstange (10) aufweist, die durch die Oeffnung (9) führt, die als Austrittsrohr hohl ausgeführt ist und die mit einem Verschluss (14) versehen ist.

8. Vorrichtung nach Anspruch 7, **dadurch gekennzeichnet, dass** ein Verschluss (14) mit einer Ausstülpung (15) die hohle Kolbenstange (10) bis zum Mischkolben (11) ausfüllt, um ein Vordringen der Pulverkomponente (2) vor und während dem Mischen zu verhindern.

9. Vorrichtung nach einem der Ansprüche 5 bis 8, **dadurch gekennzeichnet, dass** der Verdrängerkörper (12) eine Schneide (17) aufweist, mit der die Membran (3, 16) zerstörbar ist.

10. Vorrichtung nach einem der Ansprüche 1 bis 7, **dadurch gekennzeichnet, dass** der Transportraum (4) der Flüssigkomponente (1) als Ringraum in einen Behälterdeckel (7) des Behälters (6) integriert ist.

11. Vorrichtung nach einem der Ansprüche 5 bis 10, **dadurch gekennzeichnet, dass** der Verdrängerkörper (12) als axial beweglicher Ring mit dem Mischkolben (11) verschiebbar ist.

12. Vorrichtung nach einem der Ansprüche 5 bis 9, **dadurch gekennzeichnet, dass** der Transportraum (4) der Flüssigkomponente (1) als axial beweglicher Ring im Behälter (6) verschiebbar ist, während der Verdrängerkörper (12) ortsfest in einen Behälterdeckel (7) integriert ist.

13. Vorrichtung nach Anspruch 11 oder 12, **dadurch gekennzeichnet, dass** der bewegliche Ring mit einer Schnappverbindung (20) im Behälter (6) in einer Transportstellung gehalten ist, um ein ungewolltes Beschädigen der Membran (3, 16) zu verhindern.

14. Vorrichtung nach Anspruch 10 im Rückbezug auf einen der Ansprüche 1 bis 4, **dadurch gekennzeichnet, dass** der Behälterdeckel (7) mit dem Transportraum (4) der Flüssigkomponente in axialer Richtung gegenüber dem Behälter (6) beweglich ist, um die Membran (3, 16) mit einer ortsfesten Schneide (17) zu zerstören.

15. Vorrichtung nach Anspruch 14, **dadurch gekennzeichnet, dass** zwischen Schneide (17) und Transportraum (5) der Pulverkomponente (2) ein fester Ableitring (35) und ein zum Behälterdeckel (7) unter Vorspannkraft axial beweglicher Schleppring (36) angeordnet sind, welche Ringe (35, 36) die Pulverkomponente (2) vom Bereich der Schneide (17) während dem Transport und während dem Mischvorgang das Mischgut vom Bereich der Schneide (17) fernhalten, wobei mit dem ersten axialen Absenken des Behälterdeckels (7) die Membran (3, 16) in die Schneide (17) fährt und der Schleppring (36) mit dem Behälterdeckel (7) verklinkt wird, um mit dem Zurückfahren des Behälterdeckels (7) eine Ausflussöffnung (37) für die Flüssigkomponente (1) zu schaffen, welche Oeffnung (37) mit einem zweiten axialen Absenken des Behälterdeckels (7) für das Mischen wieder verschliessbar ist.

16. Vorrichtung nach Anspruch 15, **dadurch gekennzeichnet, dass** die Axialbewegung zwischen Behälterdeckel (7) und Behälter (6) durch Drehung über eine Wendel (38) erzeugt ist.

17. Vorrichtung nach einem der Ansprüche 1 bis 16, **dadurch gekennzeichnet, dass** der Behälter (6, 7) eine Absaugöffnung (21) aufweist, um Dämpfe abzusaugen.

18. Verfahren zum Mischen und Verpressen von Zweikomponentenmaterial, insbesondere von Knochenzement, mit einer Vorrichtung nach einem der Ansprüche 1 bis 17, **gekennzeichnet durch** die Schritte,
- dass der eingeschlossene feste Körper (12, 17) relativ zum Transportraum (4) der Flüssigkomponente (2) aus einer Transportstellung soweit herausbewegt wird, dass die Membrane (3, 16) zerstört wird,
- dass der Mischkolben (11) über eine bestimmte Anzahl Hübe, beispielsweise über 30 Hübe, auf- und abbewegt wird,
- dass fakultativ Lösungsmitteldämpfe aus den Transporträumen (4, 5) abgesaugt werden, und
- dass die Ausstossöffnung (9) geöffnet und der Ausstosskolben (8) betätigt wird, um die Mischung auszustossen.

19. Verfahren nach Anspruch 18, **dadurch gekennzeichnet, dass** im voraus zu Transport- und Lagerzwecken und für Knochenzement zusätzlich unter sterilen Bedingungen
• der Ausstosskolben (8) in den Behälter (6, 7) eingesetzt wird,
• der Mischkolben (11) in den Behälter (6, 7) eingesetzt wird,
• die Flüssigkomponente (1) in einen ringförmigen und mit einer Membran (3) versehbaren Transportraum (4) eingeschlossen wird,
• der Transportraum (4) der Flüssigkomponente in dem Behälter (6, 7) eingeschlossen wird,
• die Pulverkomponente (2) in den Behälter (6) eingeführt wird,
• ein fester Körper (12, 17) in den Behälter (6) eingebracht wird,
• der Behälterdeckel (7) an seiner Ausstossöffnung (9) verschlossen wird, und
• zusätzlich für Knochenzement die Vorrichtung in einer sterilen Atmosphäre in einer Transport- und Lagerfolie eingeschweisst wird.

## Claims

1. Transport and processing apparatus for a two-component material, in particular for bone cement, having a liquid component (1) and a powder component (2) which are separated by a membrane (3) and each occupy a transport chamber (4, 5) in a closeable container (6, 7) which has an expulsion piston (8) at the one end and an opening (9) for the expulsion of mixed two-component material at the opposite end and which encloses a perforated mixing piston (11), **characterised in that** the transport chamber (4) for the liquid component (1) is executed in ring shape with a central aperture (34) and with a membrane (3); **in that** the mixed two-component material can be expelled through the central aperture (34) and the opening (9); and **in that** the membrane (3) can be destroyed by a relative movement between the transport chamber (4) for the liquid component (1) and a solid body (12, 17) enclosed in the container (6) in order to effect a flow of the liquid component into the transport chamber (5) for the powder component (2).

2. Apparatus in accordance with claim 1, **characterised in that** the membrane (3) is formed by a ring-shaped film (16).

3. Apparatus in accordance with claim 1 or claim 2 **characterised in that** the transport chamber (4) for the liquid component has, in addition to the membrane (3, 16), a further filling opening (19) which can be closed off from the outside.

4. Apparatus in accordance with one of the claims 1 to 3, **characterised in that** the mixing piston (11) can be fixed axially in at least one position.

5. Apparatus in accordance with one of the claims 1 to 4, **characterised in that** a displacement member (12) is enclosed in the container (5, 6) and experiences a relative movement with respect to the transport chamber (4) for the liquid component (1) through the mixing piston (11) in order to destroy the membrane (3, 16) and to displace the liquid component (1) out of its transport chamber (4).

6. Apparatus in accordance with claim 5 **characterised in that** the displacement member (12) and the transport chamber (4) for the liquid component (1) mutually bind with one other at the end of the displacement of the liquid component (1).

7. Apparatus in accordance with one of the claims 1 to 6 **characterised in that** the mixing piston (11) has a piston rod (10) which leads through the opening (9), which is made hollow as an outlet tube and which is provided with a closure (14).

8. Apparatus in accordance with claim 7 **characterised in that** a closure (14) with a protrusion (15) fills out the hollow piston rod (10) up to the mixing piston (11) in order to prevent an intrusion of the powder component (2) prior to and during the mixing.

9. Apparatus in accordance with one of the claims 5 to 8 **characterised in that** the displacement member (12) has a cutting edge (17) by means of which the membrane (3, 16) can be destroyed.

10. Apparatus in accordance with one of the claims 1 to 7 **characterised in that** the transport chamber (4) for the liquid component (1) is integrated as a ring chamber into a container cover (7) of the container (6).

11. Apparatus in accordance with one of the claims 5 to 10 **characterised in that** the displacement member (12) can be displaced by the mixing piston (11) as an axially movable ring.

12. Apparatus in accordance with one of the claims 5 to 9 **characterised in that** the transport chamber (4) for the liquid component (1) can be displaced in the container (6) as an axially movable ring, while the displacement member (12) is integrated into the container cover (7) at a fixed location.

13. Apparatus in accordance with one of the claims 11 or 12 **characterised in that** the movable ring is held in a transport position in the container (6) by a snap connection (20) in order to prevent unintentional damage to the membrane (3, 16).

14. Apparatus in accordance with claim 10 in dependence on one of the claims 1 to 4 **characterised in that** the container cover (7) with the transport chamber (4) for the liquid component can be moved in the axial direction with respect to the container (6) in order to destroy the membrane (3, 16) with a cutting edge (17) at a fixed location.

15. Apparatus in accordance with claim 14 **characterised in that** a fixed lead off ring (35) and a draw-ring (36) which is axially movable under a bias force with respect to the container cover (7) are arranged between the cutting edge (17) and the transport chamber (5) for the powder component (2), said rings (35, 36) keeping the powder component (2) away from the region of the cutting edge (17) during the transport and keeping the material to be mixed away from the cutting edge (17) during the mixing process, with the membrane (3, 16) running into the cutting edge (17) during the first axial lowering of the container cover (7) and the draw ring (36) being latched to the container cover (7) in order to provide an outflow opening (37) for the liquid component (1) when the container cover (7) is drawn back, wherein said opening (37) can be closed off again for the mixing by a second axial lowering of the container cover (7).

16. Apparatus in accordance with claim 15 **characterised in that** the axial movement between the container cover (7) and the container (6) is produced by a rotation via a spiral (38).

17. Apparatus in accordance with one of the claims 1 to 16 **characterised in that** the container (6, 7) has a vacuum extraction opening (21) in order to suck off vapours.

18. Method for the mixing and compressing of two-component material, in particular of bone cement, using an apparatus in accordance with one of the claims 1 to 17, **characterised by** the steps,
- that the enclosed solid body (12, 17) is moved out of a transport position relative to the transport chamber (4) for the liquid component (1) to such an extent that the membrane (3, 16) is destroyed,
- that the mixing piston (11) is moved up and down over a certain number of strokes, for example over 30 strokes,
- that solvent vapours are optionally sucked off from the transport chambers (4, 5), and
- that the expulsion opening (9) is opened and the expulsion piston (8) is actuated in order to expel the mixture.

19. Method in accordance with claim 18 **characterised in that** in advance, for purposes of transport and storage, and in addition under sterile conditions for bone cement,
• the expulsion piston (8) is inserted into the container (6, 7),
• the mixing piston (11) is inserted into the container (6, 7),
• the liquid component (1) is enclosed in a ring-shaped transport chamber (4) which is provided with a membrane (3),
• the transport chamber (4) for the liquid component is enclosed in the container (6, 7),
• the powder component (2) is introduced into the container (6),
• a solid body (12, 17) is introduced into the container (6),
• the container cover (7) is closed at its expulsion opening (9), and
• in addition for bone cement, the apparatus is welded into a transport and storage film in a sterile atmosphere.

## Revendications

1. Dispositif de transport et de traitement pour un matériau à deux composants, notamment pour du ciment à os, avec un composant de liquide (1) et un composant de poudre (2) qui, en étant séparés par une membrane (3), occupent respectivement une enceinte de transport (4, 5) dans un conteneur (6, 7) pouvant être fermé, qui présente sur un côté un piston d'éjection (8) et sur le côté opposé une ouverture (9) pour l'expulsion du matériau à deux composants mélangé, et qui comprend un piston mélangeur perforé (11), **caractérisé en ce que** l'enceinte de transport (4) pour le composant de liquide (1) est réalisée en une forme annulaire avec un perçage médian (34) et avec une membrane (3), **en ce que** le matériau à deux composants mélangé peut être éjecté à travers le perçage médian (34) et l'ouverture (9) et **en ce que**, par un déplacement relatif entre l'enceinte de transport (4) du composant de liquide (1) et un corps solide (12, 17) renfermé dans le conteneur (6), la membrane (3) peut être détruite pour provoquer un écoulement du composant de liquide dans l'enceinte de transport (5) du composant de poudre (2).

2. Dispositif selon la revendication 1, **caractérisé en ce que** la membrane (3) est réalisée par une feuille annulaire (16).

3. Dispositif selon l'une des revendications 1 ou 2, **caractérisé en ce que** l'enceinte de transport (4) du composant de liquide présente, à part la membrane (3, 16), une ouverture supplémentaire de remplissage (19) pouvant être fermée de l'extérieur.

4. Dispositif selon l'une des revendications 1 à 3, **caractérisé en ce que** le piston mélangeur (11) peut être fixé axialement dans au moins une position.

5. Dispositif selon l'une des revendications 1 à 4, **caractérisé en ce qu'**un corps de refoulement (12) est renfermé dans le conteneur (6, 7) qui subit par le piston mélangeur (11) un déplacement relatif par rapport à l'enceinte de transport (4) du composant de liquide (1) pour détruire la membrane (3, 16) et pour refouler le composant de liquide (1) de son enceinte de transport (4).

6. Dispositif selon la revendication 5, **caractérisé en ce que** le corps de refoulement (12) et l'enceinte de transport (4) du composant de liquide (1) se bloquent mutuellement à la fin du refoulement du composant de liquide (1).

7. Dispositif selon l'une des revendications 1 à 6, **caractérisé en ce que** le piston mélangeur (11) présente une tige de piston (10) qui passe à travers l'ouverture (9) qui, en tant que tube de sortie, est réalisé creux et qui est pourvu d'une fermeture (14).

8. Dispositif selon la revendication 7, **caractérisé en ce qu'**une fermeture (14) avec une protubérance (15) remplit la tige de piston creuse (10) jusqu'au piston mélangeur (11) pour empêcher une avance du composant de poudre (2) avant et pendant le mélange.

9. Dispositif selon l'une des revendications 5 à 8, **caractérisé en ce que** le corps de refoulement (12) présente un tranchant (17) par lequel la membrane (3, 16) peut être détruite.

10. Dispositif selon l'une des revendications 1 à 7, **caractérisé en ce que** l'enceinte de transport (4) du composant de liquide (1) est intégrée en tant qu'espace annulaire dans un couvercle (7) du conteneur (6).

11. Dispositif selon l'une des revendications 5 à 10, **caractérisé en ce que** le corps de refoulement (12), en tant qu'anneau déplaçable axialement, est déplacable avec le piston mélangeur (11).

12. Dispositif selon l'une des revendications 5 à 9, **caractérisé en ce que** l'enceinte de transport (4) du composant de liquide (1), en tant qu'anneau déplaçable axialement, est déplaçable dans le conteneur (6) alors que le corps de refoulement (12) est intégré d'une manière fixe dans un couvercle de conteneur (7).

13. Dispositif selon la revendication 11 ou 12, **caractérisé en ce que** l'anneau mobile est retenu par une liaison à enclenchement (20) dans le conteneur dans une position de transport pour empêcher un endommagement non recherché de la membrane (3, 16).

14. Dispositif selon la revendication 10 avec référence à l'une des revendications 1 à 4, **caractérisé en ce que** le couvercle de conteneur (7) est déplaçable avec l'enceinte de transport (4) du composant de liquide dans la direction axiale par rapport au conteneur (6) pour détruire la membrane (3, 16) avec un tranchant fixe (17).

15. Dispositif selon la revendication 14, **caractérisé en ce que** sont disposées entre le tranchant (17) et l'enceinte de transport (5) du composant de poudre (2) une bague de dérivation fixe (35) et une bague traînée (36) déplaçable axialement sous précontrainte vers le couvercle de conteneur (7), ces bagues (35, 36) maintenant à distance le composant de poudre (2) de la zone du tranchant (17) pendant le transport, et pendant l'opération de mélange, le produit à mélanger est maintenu à distance de la zone du tranchant (17), où lors du premier abaissement axial du couvercle de conteneur (7), la membrane (3, 16) s'engage dans le tranchant (17) et la bague traînée (36) s'assemble par enclenchement avec le couvercle de conteneur (7) pour, avec le retour du couvercle de conteneur (7), créer une ouverture d'écoulement (37) pour le composant de liquide (1), cette ouverture (37) pouvant être fermée de nouveau par un deuxième abaissement axial du couvercle de conteneur (7) pour le mélange.

16. Dispositif selon la revendication 15, **caractérisé en ce que** le mouvement axial entre le couvercle de conteneur (7) et le conteneur (6) est produit par une rotation par une hélice (38).

17. Dispositif selon l'une des revendications 1 à 16, **caractérisé en ce que** le conteneur (6, 7) présente une ouverture d'aspiration (21) pour aspirer des vapeurs.

18. Procédé de mélange et de compression d'un matériau à deux composants, notamment d'un ciment à os, avec un dispositif selon l'une des revendications 1 à 17,
**caractérisé par** les étapes,
- que le corps fixe enfermé (12, 17), relativement à l'enceinte de transport (4) du composant de liquide (2), est sorti d'une position de transport suffisamment pour que la membrane (3, 16) soit détruite,
- que le piston mélangeur (11) est déplaçable vers le haut et vers le bas selon un nombre défini de courses, par exemple supérieur à 30 courses,
- que, d'une manière facultative, des vapeurs de solvant sont aspirées des enceintes de transport (4, 5) et
- que l'ouverture d'éjection (9) est ouverte et le piston d'éjection (8) est actionné pour expulser le mélange.

19. Procédé selon la revendication 18, **caractérisé en ce que** préalablement, à des fins de transport et de stockage, et pour du ciment à os, en plus sous des conditions stériles,
• le piston d'éjection (8) est placé dans le conteneur (6, 7),
• le piston de mélange (11) est placé dans le conteneur (6, 7),
• le composant de liquide (1) est renfermé dans une enceinte de transport annulaire (4) et pouvant être pourvue d'une membrane (3),
• l'enceinte de transport (4) du composant de liquide est renfermée dans le conteneur (6, 7),
• le composant de poudre (2) est introduit dans le conteneur (6),
• un corps fixe (12, 17) est introduit dans le conteneur (6),
• le couvercle de conteneur (7) est fermé à son ouverture d'éjection (9) et
• additionnellement pour le ciment à os, le dispositif est soudé sous une atmosphère stérile dans une feuille de transport et de stockage.
